# EUROPÄISCHE PATENTSCHRIFT

(11) **EP 0 682 655 B1**
(45) Veröffentlichungstag und Bekanntmachung des Hinweises auf die Patenterteilung: **03.05.2000**
(21) Anmeldenummer: 95902106.4
(22) Anmeldetag: 01.12.1994
(51) Int. Cl.: C07D 213/73

(54) **VERFAHREN ZUR HERSTELLUNG VON 3-AMINO-2-CHLOR-4-ALKYL- BZW. 4-CYCLOALKYL-PYRIDINE**
METHOD FOR PREPARING 3-AMINO-2-CHLORO-4-ALKYLPYRIDINE OR -4-CYCLOALKYLPYRIDINES
PROCEDE DE PREPARATION DE 3-AMINO-2-CHLORO-4-ALKYL- OU -4-CYCLOALKYLPYRIDINE

(30) Priorität: 02.12.1993 DE 4341033
(43) Veröffentlichungstag der Anmeldung: 22.11.1995
(73) Patentinhaber: Boehringer Ingelheim Pharma KG, 55216 Ingelheim am Rhein (DE); BOEHRINGER INGELHEIM INTERNATIONAL GmbH, 55218 Ingelheim am Rhein (DE)
(72) Erfinder: SCHNEIDER, Heinrich, D-55218 Ingelheim am Rhein (DE)
(74) Vertreter: Laudien, Dieter, Dr.
(86) Internationale Anmeldenummer: EP9403988
(87) Internationale Veröffentlichungsnummer: WO9515314

(56) Entgegenhaltungen:
- WO-A-92/22531
- E. KLINGSBERG 'Pyridine and its derivatives Part Three' 1962 , INTERSCIENCE PUBLISHERS , NEW YORK siehe Seite 38, Zeile 8 - Seite 38, Zeile 20
- Chem.Ber.69,2593(1936)

## Beschreibung

Die vorliegende Erfindung betrifft ein im technischen Maßstab anwendbares Verfahren zur Herstellung von 3-Amino-2-chlor-4-alkyl-pyridinen oder von 3-Amino-2-chlor-4-cycloalkylpyridinen, insbesondere zur Herstellung von 3-Amino-2-chlor-4-C₁-C₃-alkylpyridinen, oder insbesondere zur Herstellung von 3-Amino-2-chlor-4-methylpyridin.

Die genannten Alkylpyridine sind wichtige Ausgangsverbindungen zur Synthese von 5,11-Dihydro-6H-dipyrido[3,2-b : 2',3'-e]-diazepinen, besonders hervorzuheben ist das gegen HIV-Infektionen wirksame Nevirapin.

Zur Synthese von unsubstituiertem 3-Amino-2-chlorpyridin wird im Stand der Technik die Umsetzung von 3-Aminopyridin mit nascierendem Chlor vorgeschlagen (Chem. Ber. 69, 2593 (1936); Pyridine and its derivatives, part 3, Seite 38, Zeilen 8-20). Aus dem Stand der Technik sind ferner verschiedene Verfahren zur Synthese von 3-Amino-2-chlor-4-methylpyridin denkbar, beispielsweise durch katalytische Hydrierung von 2-Chlor-3-nitro-4-methyl-pyridin. 3-Amino-2-chlor-4-methylpyridin ist jedoch nicht in größeren Mengen erhältlich, da eine Chlorierung von 3-Amino-4-methylpyridin mit Chlor (WO 92/22531) im technischen Maßstab nicht durchführbar ist.

Erfindungsgemäß wird eine zweistufige Synthese, bevorzugt ohne Isolierung der ersten Stufe vorgeschlagen, bei der als Ausgangsverbindung ein entsprechendes 2,6-Dichlor-3-amino-4-alkyl-pyridin, bevorzugt das 2,6-Dichlor-3-amino-4-methylpyridin eingesetzt wird. Alternativ können die Pyridinderivate der allgemeinen Formel III, auch ausgehed von den 3-Aminopyridinen der allgemeinen Formel II, hergestellt werden. Im nachfolgenden Syntheseschema ist der Syntheseablauf erläutert:

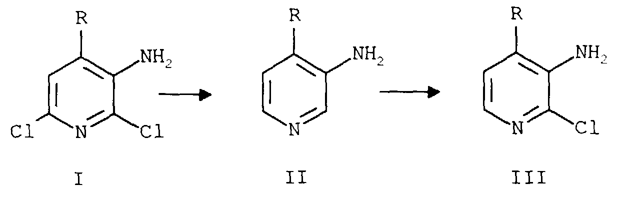

Ein Pyridinderivat der allgemeinen Formel I worin
- R =: eine Alkylgruppe mit 1 bis 8 Kohlenstoffatomen, bevorzugt C₁-C₄ Kohlenstoffatomen, ganz besonders bevorzugt Methyl,
oder
- R: eine C₃-C₆-Cycloalkylgruppe
bedeuten,
wird in einer zweistufigen Synthese durch katalytische Enthalogenierung in ein Derivat der allgemeinen Formel II, worin R die zuvorgenannte Bedeutung aufweist, überführt und anschließend durch selektive Chlorierung in 2-Position in ein Pyridinderivat der allgemeinen Formel III, worin R die zuvor genannte Bedeutung aufweist, überführt.

Als Alkylgruppen (auch soweit sie Bestandteile anderer Reste sind) werden genannt, soweit nichts anderes angegeben ist, Methyl, Ethyl, Propyl, iso-Propyl, n-Propyl, n-Butyl, iso-Butyl, sek-Butyl und tert.-Butyl.

Cycloalkyl steht im allgemeinen für einen gesättigten cyclischen Kohlenwasserstoffrest mit 3 bis 6 Kohlenstoffatomen. Als Beispiele seien Cyclopropyl, Cyclobutyl, Cyclopentyl, Cyclohexyl, genannt.

Im ersten Reaktionsschritt erfolgt die Umsetzung des Pyridins der allgemeinen Formel I durch katalytische Reduktion zu einem enthalogenierten Pyridinderivat der allgemeinen Formel II. Die bevorzugten Reduktionsmittel sind der weiteren Beschreibung und den Beispielen zu entnehmen.

Das so gebildete 3-Aminopyridinderivat der allgemeinen Formel II - worin R die in I genannte Bedeutung aufweist - kann nach üblichen Methoden aufgearbeitet und isoliert werden. Sinnvoll ist jedoch die direkte Weiterverarbeitung, die Chlorierung in der 2-Position des Pyridins unter Verwendung von HCl/H₂O₂ in wässeriger Lösung. Erfindungsgemäß geeignet ist Salzsäure, die eine Konzentration von ca. 20 Gew.-% und mehr aufweist, bevorzugt ist konzentrierte Salzsäure mit einem Gehalt von ca. 36 Gew.-%. Die Konzentration der H₂O₂-Lösung ist nicht kritisch, bevorzugt werden jedoch konzentrierte Lösungen von ca. 20 - 35 Gew.-%. Höhere Konzentrationen an H₂O₂ sind prinzipiell möglich, es ist jedoch zu beachten, daß hochkonzentrierte H₂O₂-Lösungen, wegen der Explosionsgefahr bei technischen Verfahren ein Risiko beinhalten. Die Reaktion wird üblicherweise bei Temperaturen zwischen 30 und 50°C durchgeführt.

Die weitere Aufarbeitung erfolgt durch fraktionierte Extraktion bei unterschiedlichen pH-Werten. Nach Beendigung der Reaktion wird der pH-Wert mit Natronlauge oder einer anderen geeigneten Base auf einen Wert zwischen -1 und +1 eingestellt und das Reaktionsgemisch mit einem geeigneten Lösungsmittel, bevorzugt mit einem Halogenkohlenwasserstoff extrahiert. Geeignete Lösungsmittel sind Methylenchlorid, Chloroform und andere. Geeignete Lösungsmittel sind solche, die beispielsweise gegenüber den Reaktanden beständig und aus Sicherheitsgründen schwer entflammbar sind. Man erhält nach Aufarbeitung der organischen Phase wieder entstandenes Ausgangsmaterial. In Abhängigkeit der Substituenten in der 4- und 5-Position des Pyridins der allgemeinen Formel III verringert sich die Löslichkeit - beispielsweise bei 3-Amino-2-chlor-4-n-propylpyridin- der Produkte. Diese können auch durch Ausfällen bei pH 7 - 14 aus der wässerigen Phase direkt isoliert werden. Besser lösliche Derivate - insbesondere 3-Amino-2-chlor-4-methyl-pyridin werden wie folgt aufgearbeitet: Die wässerige Phase wird auf einen pH-Wert von 3,5 bis 5 eingestellt, beispielsweise unter Verwendung einer 45 Gew-%igen Natronlauge.

Die nachfolgende Extraktion mit einem geeigneten organischen Lösungsmittel, z.B. Methylenchlorid, ergibt nach üblicher Aufarbeitung der organischen Phase das gewünschte 3-Amino-2-chlor-4-alkyl-pyridin, bevorzugt das 3-Amino-2-chlor-4-methylpyridin.

2,6-Dichlor-3-amino-4-alkylpyridin (bevorzugt R = Methyl) wird durch katalytische Reduktion in das 3-Amino-4-alkylpyridin überführt. Bevorzugtes Reduktionsmittel ist Wasserstoff in Gegenwart eines Palladiumkohle Katalysators. Bevorzugtes Lösungsmittel ist Methanol/Wasser.

Das dabei gebildete 3-Amino-4-alkylpyridin, bevorzugt das 3-Amino-4-methylpyridin, kann nach üblichen Methoden aufgearbeitet und isoliert werden. Erfindungsgemäß bevorzugt ist jedoch die direkte Weiterverarbeitung, die anschließende Chlorierung in der 2-Position unter Verwendung von HCl/H₂O₂ in wässeriger Lösung.

Die weitere Aufarbeitung erfolgt durch fraktionierte Extraktion bei unterschiedlichen pH-Werten. Nach Beendigung der Reaktion wird der pH-Wert durch Zugabe einer Base, bevorzugt Natronlauge oder einer anderen geeigneten Base, auf einen Wert zwischen -1 und +1 eingestellt und das Reaktionsgemisch mit einem Halogenkohlenwasserstoff extrahiert. Geeignete Lösungsmittel sind Methylenchlorid, Chloroform und andere.

Man erhält nach Aufarbeitung der organischen Phase das bei der Chlorierung als Nebenprodukt gebildete 2,6-Dichlor-3-amino-4-alkylpyridin, bevorzugt das 2,6-Dichlor-3-amino-4-methyl-pyridin, das wieder als Ausgangsprodukt eingesetzt werden kann.

Die wässerige Phase wird auf einen pH-Wert von 3,5 bis 5 eingestellt, beispielsweise unter Verwendung einer 45 gew-%-igen Natronlauge. Die nachfolgende Extraktion mit einem geeigneten organischen Lösungsmittel z.B. Methylenchlorid, ergibt nach üblicher Aufarbeitung der organischen Phase das gewünschte 3-Amino-2-chlor-4-alkylpyridin, bevorzugt das 3-Amino-2-chlor-4-methylpyridin.

Nach dem erfindungsgemäßen Verfahren lassen sich erstmals in einem technischen Maßstab generell 3-Amino-2-chlorpyridine herstellen, die in der 6-Position keinen Substituenten aufweisen. Die Substituenten in 4- oder in 4- und 5-Position können beliebig gewählt werden, soweit sichergestellt ist, daß sie unter den gewählten Reaktionsbedingungen beständig sind.

Ausgehend von in 4-Position substituierten oder in 4- und 5-Position substituierten 3-Amino-pyridinen erfolgt nach dem erfindungsgemäßen Verfahren die Chlorierung durch Umsetzung mit HCl/H₂O₂ fast ausschließlich in der 2-Position des Pyridins. Die Reaktionsbedingungen sind analog dem vorherbeschriebenen.

Die Ausgangsverbindungen sind entweder bekannt oder lassen sich nach einfachen Analogieverfahren herstellen.

Die nachfolgend beschriebenen Beispiele erläutern die Erfindung:

### Beispiel 1

### 1. Stufe:

### Reduktion von 2,6-Dichlor-3-amino-4-methylpyridin.

177 kg 2,6-Dichlor-3-amino-4-methylpyridin werden in 531 ltr. Methanol und 170 ltr. Wasser gelöst und mit 5 kg 10 % Palladiumkohle versetzt und bei 70 - 90°C mit Wasserstoffgas reduziert. Anschließend wird der Katalysator abfiltriert und das Lösungsmittel bis fast zur Trockne abdestilliert.

### 2. Stufe

### Chlorierung von 3-Amino-4-methylpyridin

Der so erhaltene Rückstand aus der ersten Stufe wird durch Zugabe von 250 ltr. Salzsäure (36%ig) gelöst, anschließend werden insgesamt 103 ltr. H₂O₂ (35%ig) kontinuierlich bei einer Temperatur von 35 - 45°C eingetragen. Nach Beendigung der Reaktion wird mit Natronlauge auf eine pH zwischen -1 und +1 gestellt. Anschließend wird mehrmals mit Methylenchlorid extrahiert.

Die verbleibende wässerige Phase wird dann mit Natronlauge auf einen pH zwischen 3,5 und 5 gestellt. Nach Zugabe von 6 kg Natriumsulfit wird mehrmals mit Methylenchlorid extrahiert. Die vereinten organischen Phasen werden eingeengt und aufgearbeitet.

Man erhält 122.6 kg = 86 % d. TH. des 3-Amino-2-chlor-4-methylpyridins. Durch Aufarbeitung der Mutterlaugen werden weitere 8.6 kg Produkt erhalten.

Rückgewinnung von 3-Amino-2,6-dichlor-4-methylpyridin:

Die organische Phase der ersten Extraktion oben beschriebener Synthese wird wie folgt aufgearbeitet.

Die organische Phase wird mit 6 kg Natronlauge (45%ig) und 80 ltr. Wasser versetzt und extrahiert, die untere organische Phase wird abgetrennt, die wässerige noch einmal mit 25 ltr. Methylenchlorid versetzt. Aus den vereinigten organischen Phase erhält man nach üblicher Aufarbeitung 7,1 kg = 4 % d. Th. 3-Amino-2,6-dichlor-4-methylpyridin, das wieder in die Reaktion eingesetzt werden kann.

### Beispiel 2

### 3-Amino-2-chlor-4-n-propylpyridin

In einem 1 l-Rührautoklaven werden 150.0 g 3-Amino-2,6-dichlor-4-n-propylpyridin und 5.0 g Pd-Kohle 10 % in einem Gemisch aus 450.0 ml Methanol und 150.0 ml Wasser suspensiert und bei 3 barü Wasserstoff und 50 - 70°C bis zum Ende der Gasaufnahme hydriert.

Der Katalysator wird abfiltriert und die resultierende Lösung von 3-Amino-4-n-propylpyridin x 2 HCl auf Rückstand eingeengt.

Dieser wird 219.5 g Salzsäure 36 - 37 % versetzt und dazu bei 35 - 40°C in ca. 3 h 99.5 g Wasserstoffperoxid 30 % zugetropft. Man rührt noch ca. 1,5 h bei 40°C, gibt bei 20 - 30°C 97.6 g Natronlauge 45% zu und extrahiert dreimal mit jeweils 75.0 ml Methylenchlorid. Aus diesen Extrakten können 5 - 15 % Dichlorverbindung wiedergewonnen werden. Die Wasserphase wird mit 375.0 ml Wasser verdünnt und mit ca. 227.6 g Natronlauge 45 % auf pH 12 gestellt.

Die ausgefallenen Kristalle weren abgesaugt, mit Wasser gewaschen und getrocknet.
Fp.: 75 - 77°C
Ausbeute: 98.4 g
entsprechen:78.9 % d. Th.

## Patentansprüche

1. Verfahren zur Herstellung von 3-Amino-2-chlor-4-alkylpyridinen oder 3-Amino-2-chlor-4-cycloalkyl-pyridinen, dadurch gekennzeichnet, daß man ein 3-Amino-4-alkyl-pyridin oder ein 3-Amino-4-cycloalkyl-pyridin mit HCl/H₂O₂ in wässeriger Lösung umsetzt und aufarbeitet.

2. Verfahren nach Anspruch 1, dadurch gekennzeichnet, daß man 3-Amino-4-methyl-pyridin als Ausgangsverbindung einsetzt.

3. Verfahren zur Herstellung von 3-Amino-2-chlor-4-C₁-C₃-alkylpyridinen nach Anspruch 1, dadurch gekennzeichnet, daß man
a) ein 2,6-Dichlor-3-amino-4-C₁-C₃-alkylpyridin durch katalytische Reduktion in das entsprechende 3-Amino-4-C₁-C₃-alkylpyridin überführt und anschließend
b) mit HCl/H₂O₂ in wässeriger Lösung umsetzt und aufarbeitet.

4. Verfahren nach Anspruch 3, dadurch gekennzeichnet, daß man 2,6-Dichlor-3-amino-4-methylpyridin als Ausgangsverbindung einsetzt.

5. Verfahren nach Anspruch 3 oder 4, dadurch gekennzeichnet, daß die katalytische Reduktion unter Verwendung von Wasserstoff in Gegenwart eines Palladiumkohle-Katalysators in Methanol/Wasser als Lösungsmittel durchgeführt wird.

6. Verfahren nach Anspruch 3 oder 4, dadurch gekennzeichnet, daß man das entstandene 3-Amino-4-C₁-C₃-alkylpyridin isoliert.

7. Verfahren nach einem der Ansprüche 1 bis 4, dadurch gekennzeichnet, daß die Aufarbeitung durch fraktionierte Extraktion in folgenden Schritten erfolgt:
a) Abtrennen von Nebenprodukten aus der organischen Phase in einem pH-Intervall von -1 bis +1
und
b) Extraktion der wässerigen Phase bei einem pH-Wert im Bereich von 3,5 - 5 mit einem organischen Lösungsmittel,
oder, im Fall von schwerer löslichen Derivaten, Ausfällen des Produktes aus der wässerigen Phase bei einem pH-Wert im Bereich von pH 7 - 14.

## Claims

1. Process for preparing 3-amino-2-chloro-4-alkylpyridines or 3-amino-2-chloro-4-cycloalkyl-pyridines, characterised in that a 3-amino-4-alkyl-pyridine or a 3-amino-4-cycloalkyl-pyridine is reacted with HCl/H₂O₂ in aqueous solution and worked up.

2. Process according to claim 1,characterised in that 3-amino-4-methyl-pyridine is used as the starting compound.

3. Process for preparing 3-amino-2-chloro-4-C₁₋₃-alkylpyridines according to claim 1, characterised in that
a) a 2,6-dichloro-3-amino-4-C₁₋₃-alkylpyridine is converted into the corresponding 3-amino-4-C₁₋₃-alkylpyridine by catalytic reduction and then
b) reacted with HCl/H₂O₂ in aqueous solution and worked up.

4. Process according to claim 3, characterised in that 2,6-dichloro-3-amino-4-methylpyridine is used as the starting compound.

5. Process according to claim 3 or 4, characterised in that the catalytic reduction is carried out using hydrogen in the presence of a palladium/charcoal catalyst in methanol/water as solvent.

6. Process according to claim 3 or 4, characterised in that the 3-amino-4-C₁₋₃-alkylpyridine formed is isolated.

7. Process according to one of claims 1 to 4, characterised in that the working up is carried out by fractionated extraction in the following steps:
a) separating by-products from the organic phase within a pH range of from -1 to +1
and
b) extracting the aqueous phase at a pH in the range from 3.5 - 5 with an organic solvent,
or, in the case of derivatives which are difficult to dissolve, precipitating the product from the aqueous phase at a pH in the range from pH 7 - 14.

## Revendications

1. Procédé de préparation de 3-amino-2-chloro-4-alkylpyridines ou de 3-amino-2-chloro-4-cycloalkylpyridines caractérisé en ce que l'on fait réagir une 3-amino-4-alkylpyridine ou une 3-amino-4-cycloalkylpyridine avec HCl/H₂O₂ en solution aqueuse et on la traite.

2. Procédé selon la revendication 1 caractérisé en ce que l'on utilise la 3-amino-4-méthylpyridine comme composé de départ.

3. Procédé de préparation de 3-amino-2-chloro-4-alkyle en C₁-C₃-pyridines selon la revendication 1 caractérisé en ce que
a) on convertit une 2,6-dichloro-3-amino-4-alkyle en C₁-C₃-pyridine par réduction catalytique en la 3-amino-4-alkyle en C₁-C₃-pyridine correspondante puis
b) on la fait réagir avec HCl/H₂O₂ en solution aqueuse et on la traite.

4. Procédé selon la revendication 3 caractérisé en ce que l'on utilise la 2,6-dichloro-3-amino-4-méthylpyridine comme composé de départ.

5. Procédé selon la revendication 3 ou 4 caractérisé en ce que la réduction catalytique est réalisée au moyen d'hydrogène en présence d'un catalyseur au palladium-charbon dans le méthanol/eau comme solvant.

6. Procédé selon la revendication 3 ou 4 caractérisé en ce que l'on isole la 3-amino-4-alkyle en C₁-C₃-pyridine formée.

7. Procédé selon l'une des revendications 1 à 4 caractérisé en ce que le traitement a lieu par extraction fractionnée selon les étapes suivantes:
a) séparation des sous-produits d'avec la phase organique dans un intervalle de pH de -1 à +1
et
b) extraction de la phase aqueuse à un pH dans le domaine de 3,5 - 5 avec un solvant organique, ou, dans le cas de dérivés peu solubles, précipitation du produit à partir de la phase aqueuse à un pH dans le domaine de pH 7 - 14.
